# EUROPEAN PATENT SPECIFICATION

(11) **EP 3 756 602 B1**
(45) Date of publication and mention of the grant of the patent: **20.09.2023**
(21) Application number: 20182295.4
(22) Date of filing: 25.06.2020
(51) Int. Cl.: A61B 18/14, A61B 17/29, A61B 17/3203, A61B 18/00

(54) **MULTI-FUNCTION SURGICAL INSTRUMENTS**
MULTIFUNKTIONELLE CHIRURGISCHE INSTRUMENTE
INSTRUMENTS CHIRURGICAUX MULTIFONCTIONS

(30) Priority: 27.06.2019 US 201962867292 P
(43) Date of publication of application: 30.12.2020
(73) Proprietor: Covidien LP, Mansfield, MA 02048 (US)
(72) Inventor: McHENRY, Jennifer, R., Denver, CO Colorado 80211 (US); JOSEPH, Daniel, A, Golden, CO Colorado 80401 (US)
(74) Representative: Maschio, Antonio

(56) References cited:
- US-A1- 2011 306 967
- US-A1- 2014 135 763
- US-A1- 2014 142 572
- US-A1- 2015 141 980
- US-A1- 2016 135 872
- US-A1- 2017 105 789
- US-A1- 2018 338 790

## Description

### FIELD

The present disclosure relates to surgical instruments and, more specifically, to multi-function surgical instruments.

### BACKGROUND

In minimally-invasive surgical procedures, operations are carried out within an internal body cavity through small entrance openings in the body. The entrance openings may be natural passageways of the body or may be surgically created, for example, by making a small incision into which a cannula is inserted.

Multi-function surgical instruments are beneficial in that they allow multiple surgical tasks to be performed with a single instrument, obviating the need to alternatingly remove and insert different instruments into the surgical site to perform a surgical task and/or obviating the need for simultaneously inserting multiple instruments into the surgical site to perform a surgical task.

US 2014/142572 A1 describes an electrosurgical knife having a shaft and bipolar cutting jaws at the distal end of the shaft. A tube is provided to supply irrigation liquid to the knife.

US 2014/135763 A1 describes a multifunctional surgical instrument according to the preamble of claim 1 having a housing, a shaft extending from the housing, an end effector assembly with movable jaws at the distal end of the shaft, and a movable sheath around the shaft. The sheath is movable with a monopolar electrode for blunt dissection.

US 2018/338790 A1 describes an electrocautery device having a shaft that is movable relative to a housing and a suction pipe for applying suction adjacent to the electrocautery tip.

US 2015/141980 A1 describes a surgical instrument having a housing, a shaft extending from the housing, and movable jaws at the end of the shaft. A tube disposed within the shaft applies suction to the region of the jaws.

US 2011/306967 A1 describes a surgical instrument having a housing, a shaft extending from the housing, and movable jaws at the end of the shaft. A tube disposed within the shaft applies cooling fluid to the jaws.

US 2016/135872 A1 describes surgical instruments having a housing and a monopolar end effector extending from the housing for cutting, cauterizing or sealing tissue. A translatable sheath can extend over the end effector to provide irrigation and suction.

US 2017/105789 A1 describes surgical instruments having a housing, a shaft extending from the housing, and jaws for grasping and/or cutting tissue at the distal end of the shaft. The instruments include means for providing irrigation and suction to the jaws.

### SUMMARY

As used herein, the term "distal" refers to the portion that is described which is further from a user, while the term "proximal" refers to the portion that is being described which is closer to a user. Further, any or all of the aspects described herein, to the extent consistent, may be used in conjunction with any or all of the other aspects described herein.

Provided in accordance with aspects of the present disclosure is a surgical instrument including an end effector assembly as defined in claim 1 including first and second jaw members. At least one of the first or second jaw members is movable relative to the other between a spaced-apart position and an approximated position for grasping tissue therebetween. A sheath is movable relative to the end effector assembly between a retracted position, wherein the sheath is positioned proximally of the first and second jaw members, and an extended position, wherein the sheath is disposed about the first and second jaw members. The sheath is configured to fluidly couple to a source of at least one of suction or irrigation to provide at least one of suction or irrigation at a surgical site.

The surgical instrument further includes a housing and a shaft extending distally from the housing. The end effector assembly is supported at a distal end of the shaft and the sheath is slidably disposed about the shaft.

A fluid port is disposed on the housing and configured to connect to the source of suction and/or irrigation. An internal fluid line is disposed within the housing fluidly coupling the sheath with the fluid port.

Suitably, at least one actuator disposed on the housing is selectively actuatable for deploying and retracting the sheath.

Suitably, a movable handle extends from the housing and couples to the at least one of the first or second jaw members. The movable handle is selectively actuatable to move the at least one of the first or second jaw members relative to the other.

Suitably, the sheath defines an open distal end and is configured to provide the at least one of suction or irrigation through the open distal end thereof. Alternatively or additionally, the sheath defines a plurality of apertures through a side wall thereof and is configured to provide the at least one of suction or irrigation through the plurality of apertures.

The surgical instrument further includes an energizable member coupled to the sheath and extending distally therefrom. The energizable member is configured to move with the sheath between the retracted position, wherein the energizable member is positioned proximally of the first and second jaw members, and an extended position, wherein the energizable member extends distally from the first and second jaw members.

Suitably, the energizable member is suitably configured to selectively supply monopolar energy to tissue.

The energizable member suitably defines a hook-shaped configuration or a spatula-shaped configuration.

In embodiments, the surgical instrument further includes a fluid jet member coupled to the sheath and extending distally therefrom. The fluid jet member is configured to move with the sheath between the retracted position, wherein the fluid jet member is positioned proximally of the first and second jaw members, and an extended position, wherein the fluid jet member extends distally from the first and second jaw members. The fluid jet member is configured to provide a fluid jet stream.

Suitably, at least one of the first or second jaw members is configured to connect to a source of energy for treating tissue grasped therebetween. In such embodiments, the first and second jaw members may be configured to connect to a source of bipolar electrosurgical energy for conducting energy therebetween to treat tissue grasped therebetween.

### BRIEF DESCRIPTION OF THE DRAWINGS

The above and other aspects and features of the present disclosure will become more apparent in light of the following detailed description when taken in conjunction with the accompanying drawings wherein like reference numerals identify similar or identical elements.
FIG. 1 is a perspective view of a multi-function surgical instrument in accordance with an embodiment of the present disclosure;
FIG. 2 is an enlarged, perspective view of a distal end portion of the surgical instrument of FIG. 1 wherein a deployable assembly thereof is disposed in a retracted position;
FIG. 3 is an enlarged, perspective view of the distal end portion of the surgical instrument of FIG. 1, wherein the deployable assembly thereof is disposed in a deployed position;
FIG. 4 is a perspective view of a proximal end portion of the surgical instrument of FIG. 1 with portions removed to illustrate the internal working components thereof;
FIG. 5 is an enlarged, perspective view of the distal end portion of another surgical instrument provided in accordance with an embodiment of the present invention similar to the surgical instrument of FIG. 1, wherein the deployable assembly thereof is disposed in a deployed position;
FIG. 6 is an enlarged, perspective view of the distal end portion of yet another surgical instrument provided in accordance with the present invention similar to the surgical instrument of FIG. 1, wherein the deployable assembly thereof is disposed in a deployed position;
FIG. 7 is an enlarged, perspective view of the distal end portion of still another surgical instrument provided in accordance with the present invention similar to the surgical instrument of FIG. 1, wherein the deployable assembly thereof is disposed in a deployed position;
FIG. 8 is an enlarged, perspective view of the distal end portion of still yet another surgical instrument provided in accordance with the present invention similar to the surgical instrument of FIG. 1, wherein the deployable assembly thereof is disposed in a deployed position;
FIG. 9 is a perspective view of a proximal end portion of the surgical instrument of FIG. 8 with portions removed to illustrate the internal working components thereof.

### DETAILED DESCRIPTION

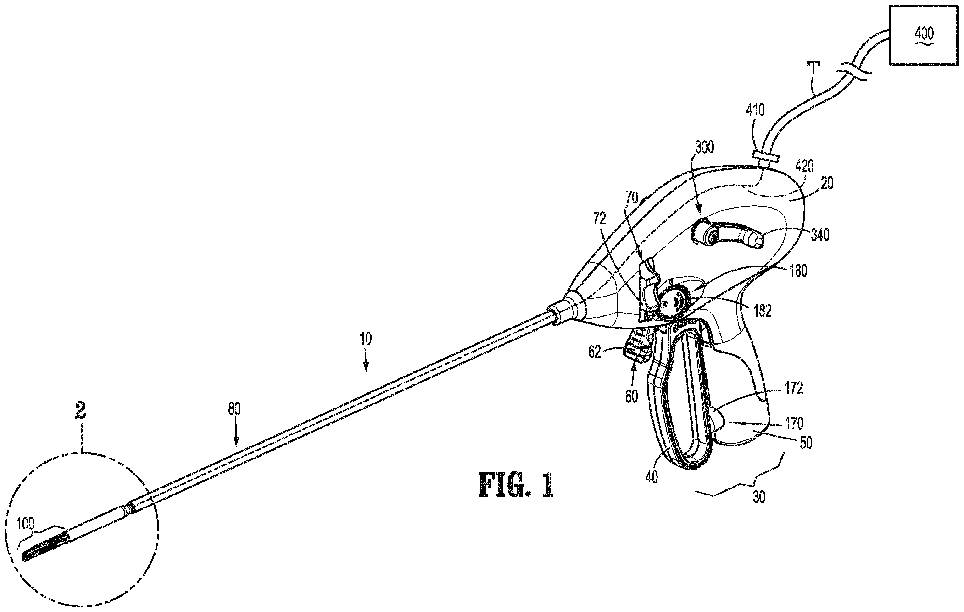

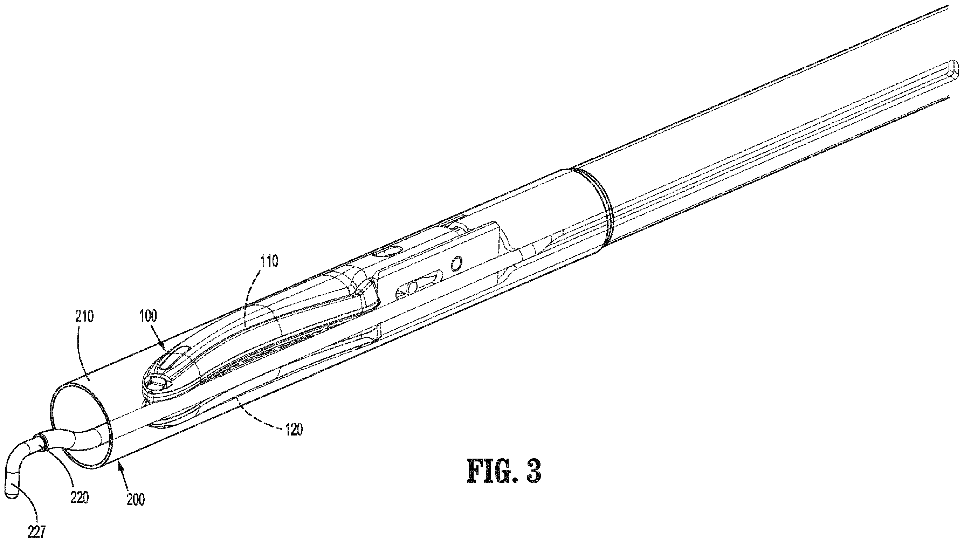

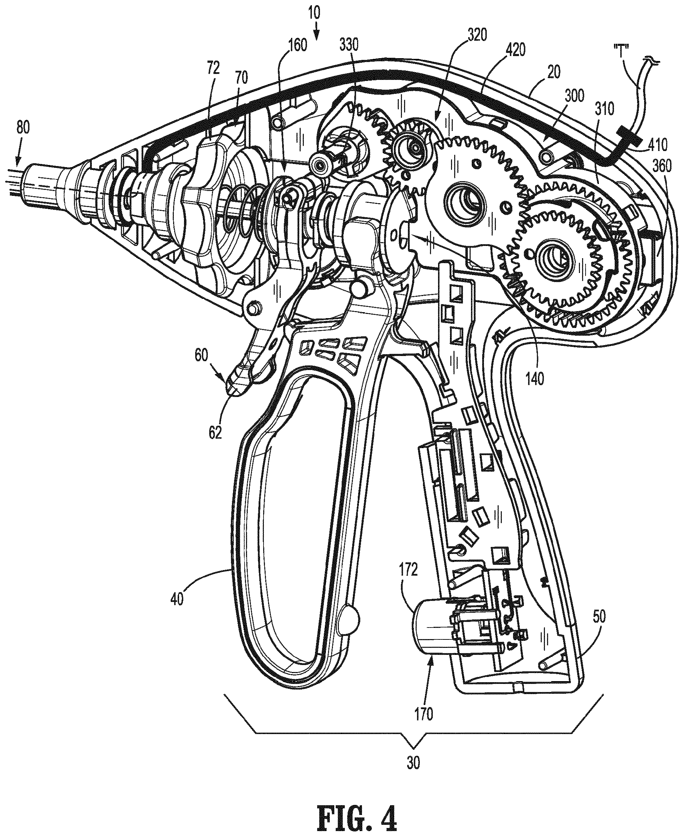

Referring generally to FIGS. 1-9, multi-function surgical instruments provided in accordance with the present invention are configured to operate in a first mode, e.g., for grasping tissue, treating grasped tissue with bipolar energy, and/or mechanically dissecting grasped tissue, and a second mode, e.g., for treating tissue and/or electrically/ electromechanically dissecting tissue with monopolar, thermal, microwave, or other suitable energy. Instrument 10 further provides suction and/or irrigation capability before, during, after, and/or in place of the first and second modes of operation.

With reference to FIGS. 1-4, a multi-function surgical instrument provided in accordance with the present invention is shown generally identified by reference numeral 10. Instrument 10 includes a housing 20, a handle assembly 30, a trigger assembly 60, a rotation assembly 70, an elongated shaft assembly 80, an end effector assembly 100, a drive assembly 140, a knife assembly 160, first and second activation assemblies 170, 180, respectively, a deployable assembly 200, and a deployment and retraction mechanism 300.

Instrument 10 also includes an electrosurgical cable (not shown) that connects instrument 10 to a generator (not shown) or other suitable power source. The electrosurgical cable includes wires (not shown) extending therethrough that have sufficient length to extend through housing 20 and/or elongated shaft assembly 80 in order to provide energy to at least one of the electrically-conductive surfaces 112, 122 of jaw members 110, 120, respectively, of end effector assembly 100, e.g., upon activation of first activation switch 172 of first activation assembly 170 in the first mode of operation. Similarly, one or more of the wires of the electrosurgical cable extends through housing 20 and/or elongated shaft assembly 80 in order to provide energy to energizable member 220 of deployable assembly 200, e.g., upon activation of either of the second activation switches 182 of second activation assembly 180 in the second mode of operation.

Instrument 10 further includes a fluid port 410 disposed on housing 20 that enables connection of instrument 10 to a suction and/or irrigation source 400 via suitable tubing "T" (integral or removable tubing "T"). Within housing 20, an internal fluid line 420 connects fluid port 410 with the interior of sheath 210 of deployable assembly 200 to enable the delivery of fluid to and/or withdrawal of fluid from the interior of sheath 210. More specifically, suction and/or irrigation source 400 may be configured to only provide suction or irrigation through sheath 210. Alternatively, suction and/or irrigation source 400 may be configured to provide, in a first configuration, suction though sheath 210, and, in a second configuration, irrigation though sheath 210. Instrument 10 may further include controls (not shown) such as ON/OFF buttons, adjustable buttons, etc. for controlling suction and/or irrigation. The controls may be disposed on housing 20, on another portion of instrument 10, or may be remote from instrument 10, e.g., on the suction and/or irrigation source 400.

Elongated shaft assembly 80 extends distally from housing 20 and supports end effector assembly 100 at a distal end thereof. End effector assembly 100 includes opposing jaw members 110, 120 pivotably coupled to one another. Each of the jaw members 110, 120 includes an electrically-conductive surface 112, 122 adapted to connect to the source of energy and defines a bipolar configuration in use wherein surface 112 is charged to a first electrical potential and surface 122 is charged to a second, different electrical potential such that an electrical potential gradient is created for conducting energy between surfaces 112, 122 and through tissue grasped therebetween for treating tissue. First activation switch 172 of first activation assembly 170 (FIG. 1) is operably coupled between the source of energy (not shown) and surfaces 112, 122 via one or more wires (not shown), thus allowing the surgeon to apply energy, e.g., bipolar electrosurgical energy, to surfaces 112, 122 of jaw members 110, 120, respectively, of end effector assembly 100 during the fist mode of operation.

Handle assembly 30 includes a movable handle 40 and a fixed handle 50. Movable handle 40 is movable relative to fixed handle 50 between an initial position, wherein movable handle 40 is spaced-apart from fixed handle 50, and a compressed position, wherein movable handle 40 is compressed towards fixed handle 50. Drive assembly 140 is operably coupled between handle assembly 30 and end effector assembly 100 such that movement of movable handle 40 between the initial position and the compressed position pivots jaw member 110 relative to jaw member 120 between the spaced-apart position and the approximated position. Bilateral configurations of jaw members 110, 120 are also contemplated.

Continuing with reference to FIGS. 1-4, trigger 62 of trigger assembly 60 is selectively actuatable relative to housing 20 from an un-actuated position to an actuated position. Knife assembly 160 is operably coupled to trigger 62 such that actuation of trigger 62 from the un-actuated position to the actuated position translates a knife (not shown) of knife assembly 160 from a retracted position, wherein knife 162 is disposed proximally of jaw members 110, 120, to an extended position, wherein knife 162 extends at least partially between jaw members 110, 120 and through knife channels (not shown) defined within jaw members 110, 120 to cut tissue grasped between jaw members 110, 120.

Rotation of rotation wheel 72 of rotation assembly 70 relative to housing 20 effects corresponding rotation of elongated shaft assembly 80, end effector assembly 100, drive assembly 140, knife assembly 160, and deployable assembly 200 relative to housing 20.

Deployable assembly 200 includes a sheath 210 and an energizable member 220. Sheath 210, in embodiments, is insulative, although other configurations are also contemplated. Sheath 210 is movable relative to end effector assembly 100 between a retracted position, wherein sheath 210 is disposed proximally of end effector assembly 100, and a extended position, wherein sheath 210 is substantially disposed about end effector assembly 100. Energizable member 220 is coupled to the source of energy (not shown) and second activation assembly 180 (FIG. 1) via one or more wires (not shown) and may function as the active electrode of a monopolar circuit or may be energizable with any other suitable form of energy, e.g., thermal, microwave, etc. Energizable member 220 is movable together with sheath 210 and relative to end effector assembly 100 between a retracted position, wherein distal tissue-treating portion 227 of energizable member 220 is positioned more-proximally, and a extended position, wherein distal tissue-treating portion 227 of energizable member 220 extends distally from end effector assembly 100 to facilitate treating tissue therewith. Energizable member 220, more specifically, is engaged with sleeve 210 such that energizable member 220 and sleeve 210 move together between their respective retracted and extended positions (collectively the retracted and extended positions of deployable assembly 200). In the extended position, in embodiments where sheath 210 is insulative, sheath 210 serves to electrically insulate end effector assembly 100 from distal tissue-treating portion 227 of energizable member 220, while distal tissue-treating portion 227 extends distally from end effector assembly 100. In the extended position, energy may be supplied to distal tissue-treating portion 227 of energizable member 220, e.g., via activation of either of the activation switches 182 of second activation assembly 180 (FIG. 1), for treating tissue in the second mode of operation.

Sheath 210, as noted above, is coupled to suction and/or irrigation source 400 via internal fluid line 420, fluid port 410, and tubing "T" to enable the delivery of fluid to and/or withdrawal of fluid from the interior of sheath 210. As such, fluid may be suctioned from a surgical site into the open distal end of sheath 210 and through sheath 210 and/or may be supplied to the surgical site via the open distal end of sheath 210. Suction and/or irrigation may be provided in the retracted position of deployable assembly 200, in the extended position of deployable assembly 200, or in both the retracted and extended positions of deployable assembly 200. Thus, suction and/or irrigation may be provided before, during, and/or after use of instrument 10 in the first mode of operation and/or second mode of operation.

Deployment and retraction mechanism 300 is configured for selectively transitioning deployable assembly 200 between its retracted position and its extended position. Deployment and retraction mechanism 300 generally includes a gear box 310 mounted within housing 20, a gear assembly 320 operably disposed within gear box 310, a pair of input shafts 330 operably coupled to gear assembly 320 and extending transversely from either side of gear box 310 and outwardly from housing 20 through apertures defined through housing 20 (only one side of housing 20 and, thus, one input shaft 330 is illustrated), a pair of deployment paddles 340 operably coupled to the input shafts 330 (only one side of housing 20 and, thus, one paddle 340 is illustrated), and a slider 360 disposed within housing 20 and operably coupling an output of gear assembly 330 with energizable member 220 of deployable assembly 200 (which, in turn, is engaged with sheath 210) such that deployment and retraction mechanism 300 is configured to enable both deployment and retraction of deployable assembly 200 in a push-push manner, e.g., wherein deployable assembly 200 is both deployed and retracted by pushing either of paddles 340 in the same direction. Other suitable deployment mechanisms are also contemplated.

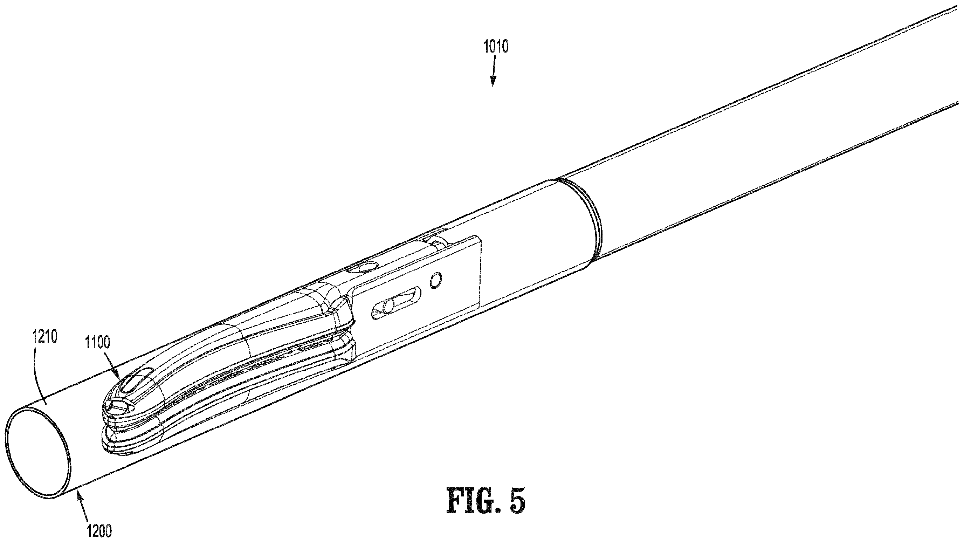

Referring to FIG. 5, another multi-function surgical instrument provided in accordance with the present invention is shown generally identified by reference numeral 1010. Instrument 1010 is similar to instrument 10 (FIG. 1) and may include any of the features thereof. Thus, for purposes of brevity, only the differences between instrument 1010 and instrument 10 (FIG. 1) are described in detail below while similarities are summarily described or omitted entirely.

Instrument 1010 is configured to operate in a first mode, e.g., for grasping tissue, treating grasped tissue with energy, and/or mechanically dissecting grasped tissue (similarly as detailed above with respect to instrument 10 (FIG. 1)), and a suction/irrigation mode, e.g., to provide suction and/or irrigation at a surgical site. More specifically, deployable assembly 1200 of instrument 1010 includes a sheath 1210 (but does not include an energizable member as with instrument 10 (FIG. 1)). Sheath 1210 is movable relative to end effector assembly 1100 between a retracted position, wherein sheath 1210 is disposed proximally of end effector assembly 1100, and a extended position, wherein sheath 1210 is substantially disposed about end effector assembly 1100.

Sheath 1210 of deployable assembly 1200 is coupled to a suction and/or irrigation source (see source 400 (FIG. 1)) to enable the delivery of fluid to and/or withdrawal of fluid from the surgical site via open distal end of sheath 1210. Suction and/or irrigation may be applied through sheath 1210 in the retracted position of sheath 1210, in the extended position of sheath 1200, or in both the retracted and extended positions of sheath 1210. Thus, suction and/or irrigation may be provided before, during, and/or after use of instrument 10 in the first mode of operation.

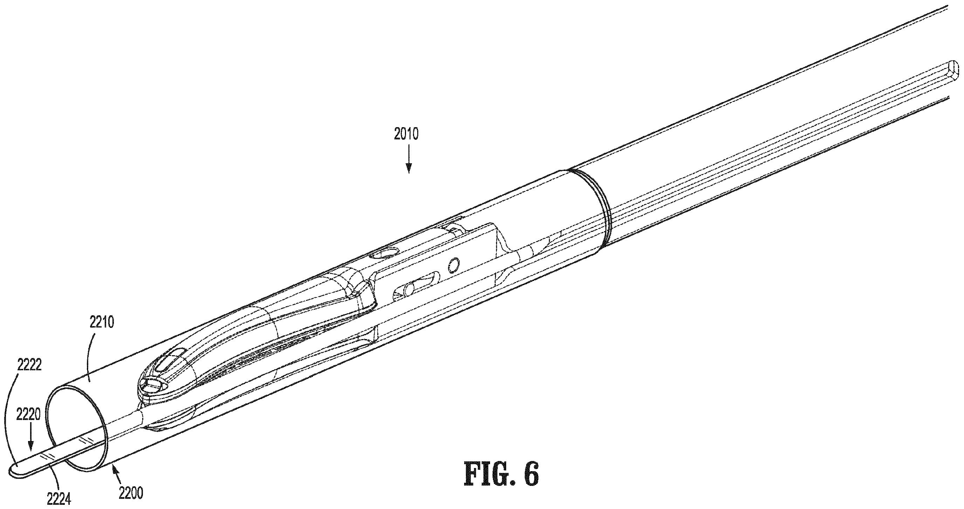

Turning to FIG. 6, another multi-function surgical instrument provided in accordance with the present invention is shown generally identified by reference numeral 2010. Instrument 2010 is similar to instrument 10 (FIG. 1) and may include any of the features thereof. Thus, for purposes of brevity, only the differences between instrument 2010 and instrument 10 (FIG. 1) are described in detail below while similarities are summarily described or omitted entirely.

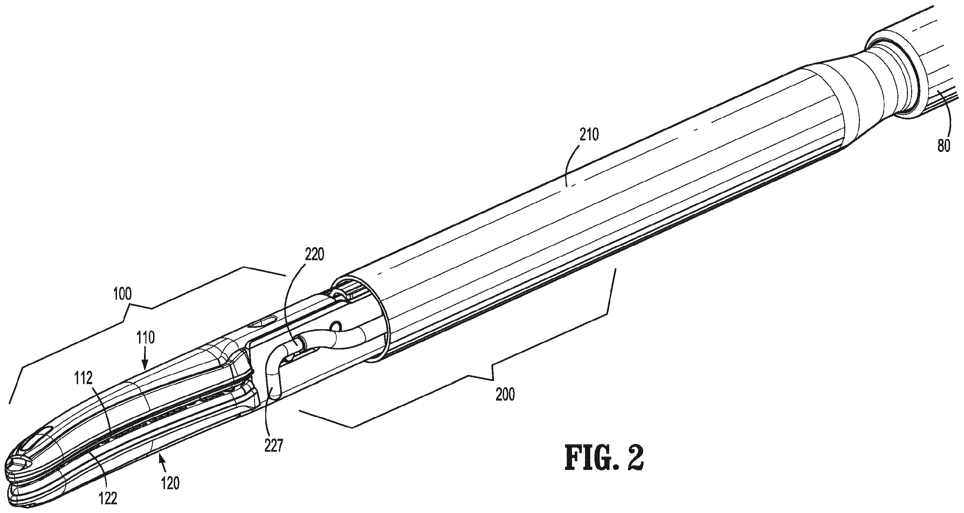

Deployable assembly 2200 of instrument 2010 includes a sheath 2210 and an energizable member 2220. Sheath 2210 of deployable assembly 2200 is coupled to a suction and/or irrigation source (see source 400 (FIG. 1)) to enable the delivery of fluid to and/or withdrawal of fluid from the interior of sheath 2210. Energizable member 2220 is coupled to the source of energy (not shown) and may function as the active electrode of a monopolar circuit or may be energizable with any other suitable form of energy, e.g., thermal, microwave, etc. In other embodiments, energizable member 2220 is not energizable (and, thus, not coupled to a source of energy) but, rather, is configured as a mechanical component configured to facilitate manual, mechanical manipulation of tissue. Energizable member 2220 defines a spatula-shaped configuration including relatively broad opposing surfaces 2222 and relatively narrow peripheral edges 2224. However, other suitable configurations (energizable or non-energizable) are also contemplated, e.g., a hook-shaped configuration (like energizable member 220 (FIG. 2)), ball-shaped configuration, needle-shaped configuration, etc.

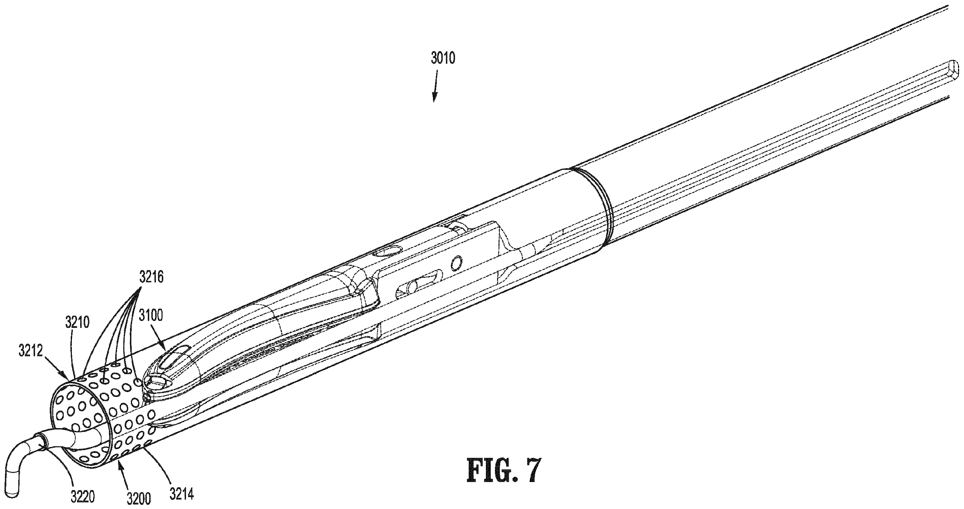

With reference to FIG. 7, another multi-function surgical instrument provided in accordance with the present invention is shown generally identified by reference numeral 3010. Instrument 3010 is similar to instrument 10 (FIG. 1) and may include any of the features thereof. Thus, for purposes of brevity, only the differences between instrument 3010 and instrument 10 (FIG. 1) are described in detail below while similarities are summarily described or omitted entirely.

Deployable assembly 3200 of instrument 3010 includes a sheath 3210 and an energizable member 3220. Energizable member 3220 may be configured similarly as energizable member 220 (FIG. 2), energizable member 2200 (FIG. 6), any other suitable energizable member, a mechanical component without energization such as those detailed herein, or may be omitted entirely. Sheath 3210 of deployable assembly 3200 includes a distal portion 3212 defining a plurality of apertures 3216 through cylindrical side wall 3214 thereof. Apertures 3216 permit passage of fluid therethrough into and out of the interior of sheath 3210. Sheath 3210 of deployable assembly 3200 is coupled to a suction and/or irrigation source (see source 400 (FIG. 1)) to enable the delivery of fluid to and/or withdrawal of fluid from the surgical site via sheath 3210, e.g., via the open distal end of sheath 3210 and/or via apertures 3216. Distal portion 3212 of sheath 3210, which includes apertures 3216, may be defined as the portion of sheath 3210 that extends distally from end effector assembly 3100 in the extended position of deployable assembly 3200, or may extend further proximally about sheath 3210. Apertures 3216 may be arranged in any suitable pattern, may extend annularly about the entire circumference of sheath 3210 or just a portion thereof, e.g., apertures 3216 may extend about 90 degrees, 180 degrees, or 270 degrees of the circumference of sheath 3210, or may intermittently be disposed about sheath 3210, e.g., to deliver or suction fluid from opposed lateral sides of sheath 3210.

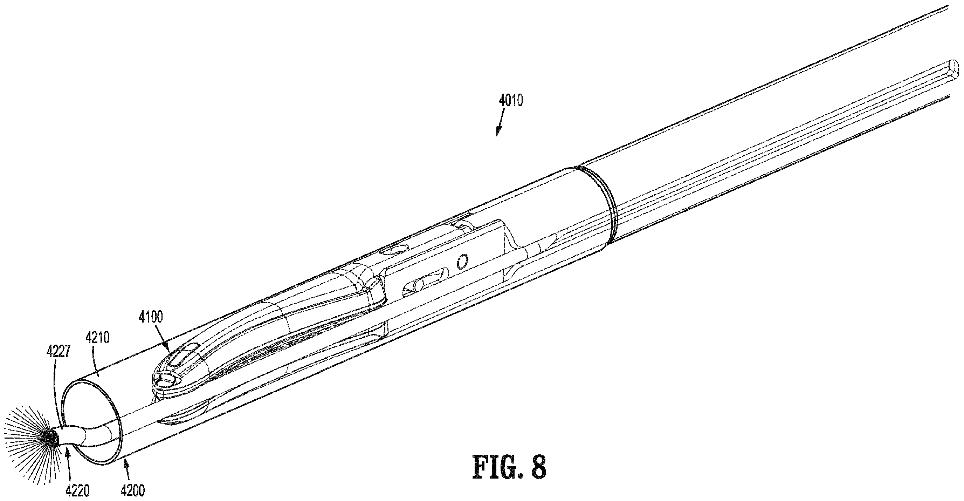

As illustrated in FIG. 8, another multi-function surgical instrument provided in accordance with the present invention is shown generally identified by reference numeral 4010. Instrument 4010 is similar to instrument 10 (FIG. 1) and may include any of the features thereof. Thus, for purposes of brevity, only the differences between instrument 4010 and instrument 10 (FIG. 1) are described in detail below while similarities are summarily described or omitted entirely.

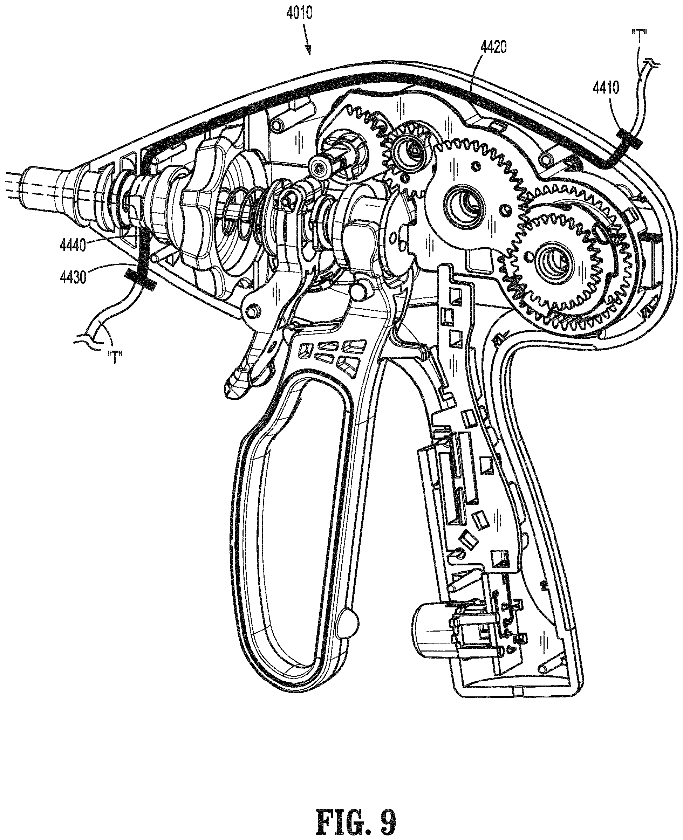

Deployable assembly 4200 of instrument 4010 includes a sheath 4210 and a fluid jet member 4220. Sheath 4210 may be configured similarly as any of the other sheaths detailed herein or in any other suitable manner. In other embodiments, sheath 4210 is omitted entirely. With additional reference to FIG. 9, sheath 4210, in embodiments where provided, is coupled to a suction source (see source 400 (FIG. 1)) via first internal fluid line 4420, first fluid port 4410, and tubing "T" to enable the withdrawal of fluid from a surgical site into the open distal end of sheath 4210 and through the interior of sheath 4210. Suction may be applied through sheath 4210 in the retracted position of deployable assembly 4200, in the extended position of deployable assembly 4200, or in both the retracted and extended positions of deployable assembly 4200. Thus, suction may be provided before, during, and/or after use of instrument 10 in the first mode of operation.

Continuing with reference to FIGS. 8 and 9, fluid jet member 4220 is movable together with sheath 4210 and relative to end effector assembly 4100 between a retracted position, wherein distal nozzle portion 4227 of fluid jet member 4220 is positioned more-proximally, and a extended position, wherein distal nozzle portion 4227 of fluid jet member 4220 extends distally from end effector assembly 2100 to facilitate supplying fluid to a surgical site. Fluid jet member 4220 is configured as fluid tube and may include distal nozzle portion 4227 to facilitate delivering fluid in the form of a jet stream, e.g., to clear blood, debris, etc. from the surgical site, to blast debris or tissue with the surgical site, and/or to cut through delicate tissue. As an alternative to a fluid jet, member 4220 may deliver fluid in any other suitable manner.

Fluid jet member 4220 is coupled to a fluid source (see source 400 (FIG. 1) or a separate source) via second internal fluid line 4440, second fluid port 4430, and tubing "T" to enable the supply of fluid to fluid jet member 4220 for application to a surgical site in the form of a fluid jet stream. Fluid jet member 4220 and/or the source may be configured to provide a variable, controllable pressure and/or flow rate of the fluid jet stream. The fluid may be water, saline, air, CO₂, medicament, cryogenic fluid, a surgical adhesive, or other suitable liquid or gas. Fluid may be supplied from fluid jet member 4220 in the retracted position of deployable assembly 4200, in the extended position of deployable assembly 4200, or in both the retracted and extended positions of deployable assembly 4200. Thus, fluid may be provided before, during, and/or after use of instrument 10 in the first mode of operation.

Turning back to FIG. 1, as opposed to handheld, manual manipulation and operation, the various embodiments disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

From the foregoing and with reference to the various drawings, those skilled in the art will appreciate that certain modifications can be made to the presently disclosed embodiments without departing from the scope of the accompanying claims.

## Claims

1. A surgical instrument (10, 1010, 2010, 3010, 4010), comprising:
a housing (20);
a shaft (80) extending distally from the housing (20);
an end effector assembly (100, 1100, 3100, 4100) supported at the distal end of the shaft (80) and including first and second jaw members (110, 120), at least one of the first or second jaw members movable relative to the other between a spaced-apart position and an approximated position for grasping tissue therebetween;
a sheath (210, 1210, 2210, 3210, 4210) slidably disposed about the shaft (80) and movable relative to the end effector assembly between a retracted position, wherein the sheath is positioned proximally of the first and second jaw members (110, 120), and an extended position, wherein the sheath is disposed about the first and second jaw members; and
an energizable member (220, 2220, 3220) coupled to the sheath (210, 2210, 3210) and extending distally therefrom, the energizable member configured to move with the sheath between the retracted position, wherein the energizable member is positioned proximally of the first and second jaw members (110, 120), and an extended position, wherein the energizable member extends distally from the first and second jaw members,
**characterized in that** the surgical instrument further comprises:
a fluid port (410) disposed on the housing (20), the fluid port configured to connect to a source of at least one of suction or irrigation; and
an internal fluid line (420) disposed within the housing (20) and fluidly coupling the sheath (210) with the fluid port (410),
whereby the sheath (210, 1210, 2210, 3210, 4210) is configured to fluidly couple to a source of at least one of suction or irrigation to provide at least one of suction or irrigation at a surgical site.

2. The surgical instrument according to claim 1, further comprising at least one actuator (340) disposed on the housing (20), the at least one actuator coupled to the sheath (210, 1210, 2210, 3210, 4210) and selectively actuatable for deploying and retracting the sheath.

3. The surgical instrument according to claim 2, further comprising a movable handle (40) extending from the housing (20) and coupled to the at least one of the first or second jaw members (110, 120), the movable handle selectively actuatable to move the at least one of the first or second jaw members relative to the other.

4. The surgical instrument according to any of claims 1 to 3, wherein the sheath (210, 1210, 2210, 3210, 4210) defines an open distal end and is configured to provide the at least one of suction or irrigation through the open distal end thereof.

5. The surgical instrument according to any of claims 1 to 3, wherein the sheath (3210) defines a plurality of apertures (3216) through a side wall thereof and is configured to provide the at least one of suction or irrigation through the plurality of apertures.

6. The surgical instrument according to any preceding claim, wherein the energizable member (220, 2220, 3220) is configured to selectively supply monopolar energy to tissue.

7. The surgical instrument according to any preceding claim, wherein the energizable member (220, 2220, 3220) defines a hook-shaped configuration or a spatula-shaped configuration.

8. The surgical instrument according to claim 1, further comprising a fluid jet member (4220) coupled to the sheath (4210) and extending distally therefrom, the fluid jet member configured to move with the sheath between the retracted position, wherein the fluid jet member is positioned proximally of the first and second jaw members (110, 120), and an extended position, wherein the fluid jet member extends distally from the first and second jaw members, the fluid jet member configured to provide a fluid jet stream.

9. The surgical instrument according to any preceding claim, wherein at least one of the first or second jaw members (110, 120) is configured to connect to a source of energy for treating tissue grasped therebetween.

10. The surgical instrument according to claim 9, wherein the first and second jaw members (110, 120) are configured to connect to a source of bipolar electrosurgical energy for conducting energy therebetween to treat tissue grasped therebetween.

## Patentansprüche

1. Chirurgisches Instrument (10, 1010, 2010, 3010, 4010), umfassend:
ein Gehäuse (20);
eine Welle (80), die sich von dem Gehäuse (20) distal erstreckt;
eine Endeffektoranordnung (100, 1100, 3100, 4100), die an dem distalen Ende der Welle (80) getragen wird und erste und zweite Backenelemente (110, 120) einschließt, wobei mindestens eines des ersten oder des zweiten Backenelements relativ zu dem anderen zwischen einer beabstandeten Position und einer angenäherten Position zum Erfassen von Gewebe dazwischen bewegbar ist;
eine Hülle (210, 1210, 2210, 3210, 4210), die verschiebbar um die Welle (80) herum angeordnet und relativ zu der Endeffektoranordnung zwischen einer eingefahrenen Position bewegbar ist, wobei die Hülle proximal des ersten und zweiten Backenelements (110, 120) und einer erstreckten Position positioniert ist, wobei die Hülle um das erste und das zweite Backenelement herum angeordnet ist; und
ein erregbares Element (220, 2220, 3220), das mit der Hülle (210, 2210, 3210) gekoppelt ist und sich distal davon erstreckt, wobei das erregbare Element konfiguriert ist, um sich mit der Hülle zwischen der eingefahrenen Position zu bewegen, wobei das erregbare Element von dem ersten und dem zweiten Backenelement (110, 120) und einer erstreckten Position proximal positioniert ist, wobei sich das erregbare Element von dem ersten und dem zweiten Backenelement distal erstreckt,
**dadurch gekennzeichnet, dass** das chirurgische Instrument ferner umfasst:
einen Fluidanschluss (410), der auf dem Gehäuse (20) angeordnet ist, wobei der Fluidanschluss konfiguriert ist, um mit einer Quelle von mindestens einem von einer Absaugung oder einer Spülung zu verbinden; und
eine interne Fluidleitung (420), die innerhalb des Gehäuses (20) angeordnet ist und die Hülle (210) mit dem Fluidanschluss (410) fluidisch koppelt,
wobei die Hülle (210, 1210, 2210, 3210, 4210) konfiguriert ist, um mit einer Quelle von mindestens einem von der Absaugung oder der Spülung fluidisch zu koppeln, um mindestens eines von der Absaugung oder der Spülung an einer Operationsstelle bereitzustellen.

2. Chirurgisches Instrument nach Anspruch 1, ferner umfassend mindestens einen Aktuator (340), der an dem Gehäuse (20) angeordnet ist, wobei der mindestens eine Aktuator mit der Hülle (210, 1210, 2210, 3210, 4210) gekoppelt ist und zum Einsetzen und Einfahren der Hülle selektiv betätigbar ist.

3. Chirurgisches Instrument nach Anspruch 2, ferner umfassend einen bewegbaren Griff (40), der sich von dem Gehäuse (20) erstreckt und mit dem mindestens einen der ersten oder zweiten Backenelemente (110, 120) gekoppelt ist, wobei der bewegbare Griff selektiv betätigbar ist, um das mindestens eine der ersten oder zweiten Backenelemente relativ zu dem anderen zu bewegen.

4. Chirurgisches Instrument nach einem der Ansprüche 1 bis 3, wobei die Hülle (210, 1210, 2210, 3210, 4210) ein offenes distales Ende definiert und konfiguriert ist, um das mindestens eine von der Absaugung oder der Spülung durch das offene distale Ende davon hindurch bereitzustellen.

5. Chirurgisches Instrument nach einem der Ansprüche 1 bis 3, wobei die Hülle (3210) eine Vielzahl von Öffnungen (3216) durch eine Seitenwand davon hindurch definiert und konfiguriert ist, um das mindestens eine von der Absaugung oder der Spülung durch die Vielzahl von Öffnungen hindurch bereitzustellen.

6. Chirurgisches Instrument nach einem der vorstehenden Ansprüche, wobei das erregbare Element (220, 2220, 3220) konfiguriert ist, um eine monopolare Energie in Gewebe selektiv zuzuführen.

7. Chirurgisches Instrument nach einem der vorstehenden Ansprüche, wobei das erregbare Element (220, 2220, 3220) eine hakenförmige Konfiguration oder eine spatelförmige Konfiguration definiert.

8. Chirurgisches Instrument nach Anspruch 1, ferner umfassend ein Fluidstrahlelement (4220), das mit der Hülle (4210) gekoppelt ist und sich distal davon erstreckt, wobei das Fluidstrahlelement konfiguriert ist, um sich mit der Hülle zwischen der eingefahrenen Position zu bewegen, wobei das Fluidstrahlelement von dem ersten und dem zweiten Backenelement (110, 120) und einer erstreckten Position proximal positioniert ist, wobei sich das Fluidstrahlelement von dem ersten und dem zweiten Backenelement distal erstreckt, wobei das Fluidstrahlelement konfiguriert ist, um einen Fluidstrahlstrom bereitzustellen.

9. Chirurgisches Instrument nach einem der vorstehenden Ansprüche, wobei mindestens eines des ersten oder des zweiten Backenelements (110, 120) konfiguriert ist, um mit einer Energiequelle zum Behandeln von dazwischen erfasstem Gewebe verbunden zu werden.

10. Chirurgisches Instrument nach Anspruch 9, wobei das erste und das zweite Backenelement (110, 120) konfiguriert sind, um mit einer Quelle von bipolarer elektrochirurgischer Energie zum Leiten von Energie dazwischen zu verbinden, um dazwischen erfasstes Gewebe zu behandeln.

## Revendications

1. Instrument chirurgical (10, 1010, 2010, 3010, 4010), comprenant :
un logement (20) ;
un arbre (80) s'étendant distalement à partir du logement (20) ;
un ensemble effecteur d'extrémité (100, 1100, 3100, 4100) supporté au niveau de l'extrémité distale de la tige (80) et comportant des premier et second éléments de mâchoire (110, 120), au moins l'un des premier ou second éléments de mâchoire pouvant être déplacé par rapport à l'autre entre une position espacée et une position rapprochée pour saisir un tissu entre eux ;
une gaine (210, 1210, 2210, 3210, 4210) disposée de manière coulissante autour de l'arbre (80) et mobile par rapport à l'ensemble effecteur d'extrémité entre une position rétractée, dans lequel la gaine est positionnée de manière proximale par rapport aux premier et second éléments de mâchoire (110, 120), et une position déployée, dans lequel la gaine est disposée autour des premier et second éléments de mâchoire ; et
un élément excitable (220, 2220, 3220) accouplé à la gaine (210, 2210, 3210) et s'étendant de manière distale à partir de celle-ci, l'élément excitable étant conçu pour se déplacer avec la gaine entre la position rétractée, dans lequel l'élément excitable est positionné de manière proximale par rapport aux premier et second éléments de mâchoire (110, 120), et une position déployée, dans lequel l'élément excitable s'étend distalement à partir des premier et second éléments de mâchoire,
**caractérisé en ce que** l'instrument chirurgical comprend en outre :
un orifice de fluide (410) disposé sur le logement (20), l'orifice de fluide étant conçu pour se raccorder à une source d'au moins l'un parmi une aspiration ou une irrigation ; et
une conduite de fluide interne (420) disposée à l'intérieur du logement (20) et couplant fluidiquement la gaine (210) à l'orifice de fluide (410),
moyennant quoi la gaine (210, 1210, 2210, 3210, 4210) est conçue pour se coupler fluidiquement à une source d'au moins l'une parmi une aspiration ou une irrigation pour fournir au moins l'une parmi une aspiration ou une irrigation au niveau d'un site chirurgical.

2. Instrument chirurgical selon la revendication 1, comprenant en outre au moins un actionneur (340) disposé sur le logement (20), l'au moins un actionneur étant accouplé à la gaine (210, 1210, 2210, 3210, 4210) et actionnable de manière sélective pour déployer et rétracter la gaine.

3. Instrument chirurgical selon la revendication 2, comprenant en outre une poignée mobile (40) s'étendant à partir du logement (20) et accouplée à l'au moins un des premier ou second éléments de mâchoire (110, 120), la poignée mobile pouvant être actionnée sélectivement pour déplacer l'au moins un des premier ou second éléments de mâchoire par rapport à l'autre.

4. Instrument chirurgical selon l'une quelconque des revendications 1 à 3, dans lequel la gaine (210, 1210, 2210, 3210, 4210) définit une extrémité distale ouverte et est conçue pour fournir l'au moins une parmi une aspiration ou une irrigation à travers l'extrémité distale ouverte de celle-ci.

5. Instrument chirurgical selon l'une quelconque des revendications 1 à 3, dans lequel la gaine (3210) définit une pluralité d'ouvertures (3216) à travers une paroi latérale de celle-ci et est conçue pour fournir l'au moins une d'une aspiration ou d'une irrigation à travers la pluralité d'ouvertures.

6. Instrument chirurgical selon l'une quelconque des revendications précédentes, dans lequel l'élément excitable (220, 2220, 3220) est conçu pour fournir sélectivement de l'énergie monopolaire au tissu.

7. Instrument chirurgical selon l'une quelconque des revendications précédentes, dans lequel l'élément excitable (220, 2220, 3220) définit une configuration en forme de crochet ou une configuration en forme de spatule.

8. Instrument chirurgical selon la revendication 1, comprenant en outre un élément de jet de fluide (4220) accouplé à la gaine (4210) et s'étendant de manière distale à partir de celui-ci, l'élément de jet de fluide étant conçu pour se déplacer avec la gaine entre la position rétractée, dans lequel l'élément de jet de fluide est positionné de manière proximale par rapport aux premier et second éléments de mâchoire (110, 120), et une position déployée, dans lequel l'élément de jet de fluide s'étend distalement à partir des premier et second éléments de mâchoire, l'élément de jet de fluide étant conçu pour fournir un courant de jet de fluide.

9. Instrument chirurgical selon l'une quelconque des revendications précédentes, dans lequel au moins l'un des premier ou second éléments de mâchoire (110, 120) est conçu pour se raccorder à une source d'énergie afin traiter le tissu saisi entre eux.

10. Instrument chirurgical selon la revendication 9, dans lequel les premier et second éléments de mâchoire (110, 120) sont conçus pour se raccorder à une source d'énergie électrochirurgicale bipolaire pour conduire de l'énergie entre eux afin de traiter le tissu saisi entre eux.
